(19) Europäisches Patentamt
European Patent Office
Office européen des brevets

(11) Publication number: **0 569 598 A1**

(12) **EUROPEAN PATENT APPLICATION published in accordance with Art. 158(3) EPC**

(21) Application number: **92924017.4**

(22) Date of filing: **27.11.92**

(86) International application number:
**PCT/JP92/01550**

(87) International publication number:
**WO 93/11121 (10.06.93 93/14)**

(51) Int. Cl.5: **C07D 265/22**, C07D 413/12, C07D 413/14, C07D 417/12, A61K 31/535

(30) Priority: **28.11.91 JP 337901/91**

(43) Date of publication of application:
**18.11.93 Bulletin 93/46**

(84) Designated Contracting States:
**AT BE CH DE DK ES FR GB GR IE IT LI LU MC NL PT SE**

(71) Applicant: **JAPAN TOBACCO INC.**
**12-16, Higashi Shinagawa 4-chome,**
**Shinagawa-ku, Tokyo 140(JP)**

(72) Inventor: **KOBAYASHI, K., Pharmac. Research Lab.**
**of Japan Tobacco Inc.**
**6-2, Umegaoka**
**Midori-ku**
**Yokohama-shi, Kanagawa-ken 227(JP)**
Inventor: **MANABE, Sh., Pharmac. Research Lab.**
**of Japan Tobacco Inc.**
**6-2, Umegaoka**
**Midori-ku**
**Yokohama-shi, Kanagawa-ken 227(JP)**
Inventor: **WATANABE, Y., Pharmac. Research Lab.**
**of Japan Tobacco Inc.**
**6-2, Umegaoka**
**Midori-ku**
**Yokohama-shi, Kananagawa-ken 227(JP)**
Inventor: **HAYAKAWA, K., Pharmac. Research Lab.**
**of Japan Tobacco Inc.**
**6-2, Umegaoka**
**Midori-ku**
**Yokohama-shi, Kanagawa-ken 227(JP)**
Inventor: **UCHIDA, I., Pharmac. Research Lab.**
**of Japan Tobacco Inc.**
**6-2, Umegaoka**
**Midori-ku**
**Yokohama-shi, Kanagawa-ken 227(JP)**

(74) Representative: **Reinhard, Horst, Dr. et al**
**Patentanwälte**
**REINHARD . SKUHRA . WEISE,**
**Postfach 44 01 51**
**D-80750 München (DE)**

(54) **BENZOXAZINONE DERIVATIVE.**

(57) A benzoxazinone derivative represented by general formula (I) or a pharmaceutically acceptable acid addition salt thereof, and an anti-inflammatory, a neutrophil infiltration inhibitor and a serine protease inhibitor each containing the derivative as an active ingredient, wherein R, $R^1$, $R^2$ and $R^3$ may be the same or different from one another and each represents hydrogen or lower alkyl; A represents optionally halogenated phenyl, heterocycle or adamantyl; B represents hydrogen or optionally halogenated phenyl or heterocycle; U represents =CH-, =C= or =N-; V represents - O-, $-CH_2-$, =N-, -NH-, -CH= or a single bond; X represents -O-, =CH-, $-CH_2-$ or a single bond; Y represents - $CH_2-$, -NH-, -O- or a single bond; Z represents -CO- or $-CH_2-$; and l represents an integer of 0 to 3. This compound has excellent activities of inhibiting serine protease, neutrotaxis,

EP 0 569 598 A1

and infiltration of neutrophils into carragheenin-induced pneumocephalus.

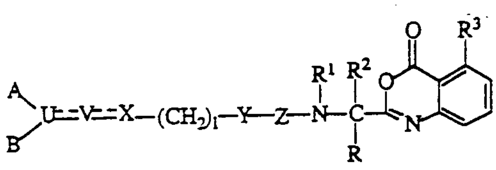

(I)

[Technical Field]

The present invention relates to a benzoxazinone derivative which has an anti-inflammatory activity, a neutrophile infiltration-suppressing activity or a serine protease inhibitory activity and which is therefore useful in the medicinal field.

[Background Art]

Infiltration of immunocompetent cells, particularly neutrophiles, from blood into tissue, which characterizes the basic morbidity of inflammation, largely involves in the formation of edema subsequently caused by leakage of blood components and to the progress of inflammatory symptoms accompanying with the tissue destruction. The abnormality of immunity is considered to promote inflammatory symptoms in various diseases. Among these diseases are: chronic rheumatoid arthritis; diseases of respiratory organs such as bronchitis based on chronic respiratory tract infection, adult respiratory distress syndrome, bronchi-obstructive type asthma classified as adult type asthma; colon disease which is one of intestinal diseases; and non-specific inflammatory diseases such as psoriasis which is one of dermatitis.

A protease, in particular, elastase, other than various inflammatory cytokines, chemical mediators, and the like produced by a neutrophile digests fiber proteins such as elastin and collagen which constitutes interstitial connective tissues present in the lungs, cartilage, blood-vessel walls, and skin etc., of higher animals, and possesses cytopathogenic activity etc. Hence, the protease plays a significant role in an etiological relationship between the formation of edema as well as the induction of inflammatory symptoms accompanying with the tissue destruction and infiltration of neutrophiles. Accordingly, an elastase inhibiting agent is considered effective as a prophylactic and therapeutic agent for the above-described diseases, diseases in organs including pancreatitis, nephritis, arteriosclerosis etc., and various diseases including septicemia etc., which attributed to destruction and deterioration of tissue and cytopathy caused by elastase.

Heretofore, several peptide-type and on-peptide-type compounds have been reported as serine protease inhibitors including elastase inhibitors. For example, non-peptide-type inhibitors are reported in "Journal of the Biochemistry", Vol. 257, Pages 5085 - 5091 (1982), "Journal of the Medicinal Chemistry", Vol. 30, Pages 1017 - 1023 (1987), " Journal of the Medicinal Chemistry", Vol. 31, Pages 1052 - 1061 (1988), "Journal of the Medicinal Chemistry", Vol. 33, Pages 464 - 479, (1990), Published Unexamined Japanese Patent Application No. 1-308227, WO 088/9790, EPO 337549, and the like.

However, in a local inflammatory region, endogenous protease inhibitors are co-present with proteases in a large amount. This fact suggests that the presence of the protease inhibiting activity alone is not sufficient to prevent and suppress the initiation and the progression of the inflammation. For example, in the hypothesis on chronic rheumatoid arthritis proposed by Lower et al. {Journal of Rheumatol, Vol. 14, Page 49 (1987)}, the neutrophiles, once infiltrate into car-tilage, etc., is considered to cause tissue destruction in the cartilage, etc., without being interfered their protease activity with endogenous protease inhibitors. From the foregoing, it is now desired and important to develop a medicine which can inhibits both the neutrophile infiltration followed by the cell deposition and the migration of, and the tissue destruction caused by the elastase activity derived from the infiltrated neutrophiles.

[Disclosure of the Invention]

The present inventors conducted intensive and extensive studies, with a view toward satisfying the above-mentioned desire to develop a medicine having the activity to effect on the wide range of the process to the initiation of inflammation and suppressing tissue destruction. They found a benzoxazinone derivative represented by a formula[I], having the excellent inhibiting activity relative to serine protease, in particular, elastase, serving to suppress chemotaxis of neutrophiles in the human peripheral blood toward chemical attractants derived from bacteria and further suppressing neutrophile infiltration in an animal inflammation model. Based on the above findings, is accomplished the present invention.

Specifically, the first object of the present invention is to provide a benzoxazinone derivative represented by the following formula [I] or a pharmaceutically acceptable acid-addition salt thereof:

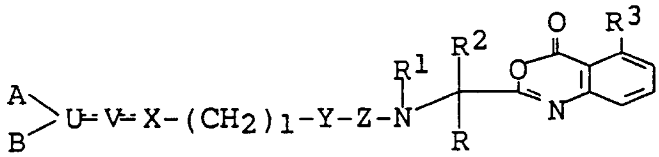

[I]

Wherein:

R is a hydrogen atom or a lower alkyl group;

$R^1$ is a hydrogen atom or a lower alkyl group;

$R^2$ is a hydrogen atom or a lower alkyl group;

$R^3$ is a hydrogen atom or a lower alkyl group;

A is a phenyl group which may be substituted with a halogen atom, or a heterocyclic ring, or adamantyl group;

B is a hydrogen atom, or a phenyl group which may be substituted with a halogen atom or a heterocyclic ring:

U is

$>$CH-,

$>$C=

or $>$N-

V is -O-, $-CH_2-$, =N-, -NH- or -CH=, or means that U is directly bond to X;

X is -O-, =CH- or $-CH_2-$, or means that V is directly bond to $(CH_2)_l$;

Y is $-CH_2-$, -NH- or -O-, of means that $(CH_2)_l$ is directly bond to N;

Z is -CO- or $-CH_2-$;

-----

is a double bond or a single bond;

1 is an integer of 0 to 3.

The second object of the present invention is to provide an anti-inflammatory agent, a neutrophile infiltration inhibiting agent, and a serine protease inhibiting agent containing the above-described benzoxazinone derivative represented by a formula [I] as an active ingredient.

The definition of terminology used in the description of this specification and examples are as follows:

"lower alkyl group" is a straight or branched alkyl group having 1 to 5 carbon atoms. As examples of the lower alkyl group, there can be mentioned a methyl group, an ethyl group, a propyl group, an isopropyl group, a butyl group, an isobutyl group, a sec-butyl group, a tert-butyl group, a pentyl group, an isopentyl group, and the like.

"Halogen atom" is fluorine, chlorine, bromine, or iodine.

"A phenyl group which may be substituted with a halogen atom" is a phenyl group which may be substituted with 1 to 3 of the above-described halogen atom. When the phenyl group is substituted with a plurality of halogen atoms, those halogen atoms can be the same or different. Examples include 4-fluorophenyl, 3-fluorophenyl, 2-fluorophenyl, 4-chlorophenyl, 3-chlorophenyl, 2-chlorophenyl, 4-bromophenyl, 3-bromophenyl, 2-bromophenyl,4-iodophenyl, 3-iodophenyl, 2-iodophenyl, 2,4-difluorophenyl, 2,4-dichlorophenyl, 2,4-dibromophenyl, 2,4-diiodophenyl, 2,4,6-trifluorophenyl, 2,4,6-trichlorophenyl, 2,4,6-tribromophenyl, 2,4,6-triiodophenyl, and the like.

"Heterocyclic ring" is a 4- to 7-membered ring having 1 or a plurality of hetero atoms. Examples include pyrrole, furan, thiophene, pyrazole, isoxazole, imidazole, oxazole, thiazole, pyridine, pyrimidine, pyrazine, azethidine, pyrrolidine, tetrahydrofuran, piperidine, piperazine, morphorine, homopiperidine, and the like, which can be bonded at any position of the heterocyclic ring.

According to the present invention, the benzoxazinone derivative represented by a general formula [I] can be produced in accordance with the following flow 1 to 3, which should not be construed as limiting the method for producing the target compound [I] of the present invention.

The definition of A, B, U, V, X, Y, Z, R, $R^1$, $R^2$, $R^3$, and 1 is the same as in the above. $L^1$ is a hydrogen atom or a carboxyl-protecting group such as an ethyl group or a benzyl group, which is usually used in peptide synthesis. $L^2$ is an amino-protecting group such as a benzyloxycarbonyl group or tert-butoxycarbonyl group, which is usually used in peptide synthesis.

Each process in reactions (a) to (d) and (g) shown in the flows 1 to 3 described below is basically a reaction for peptide bond formation. The known method usually used in peptide synthesis can be appropriately employed (for example, "Fundamentals and experiments in peptide synthesis", written by Nobuo Izumiya, published by Maruzen Co., Ltd.). Reaction (e), (f) and (h), and (i) are performed by way of a ring closure reaction using a known dehydrating agent, a known reductive alkylation reaction and a known alkylation reaction, respectively.

flow-1

Z is -CO-

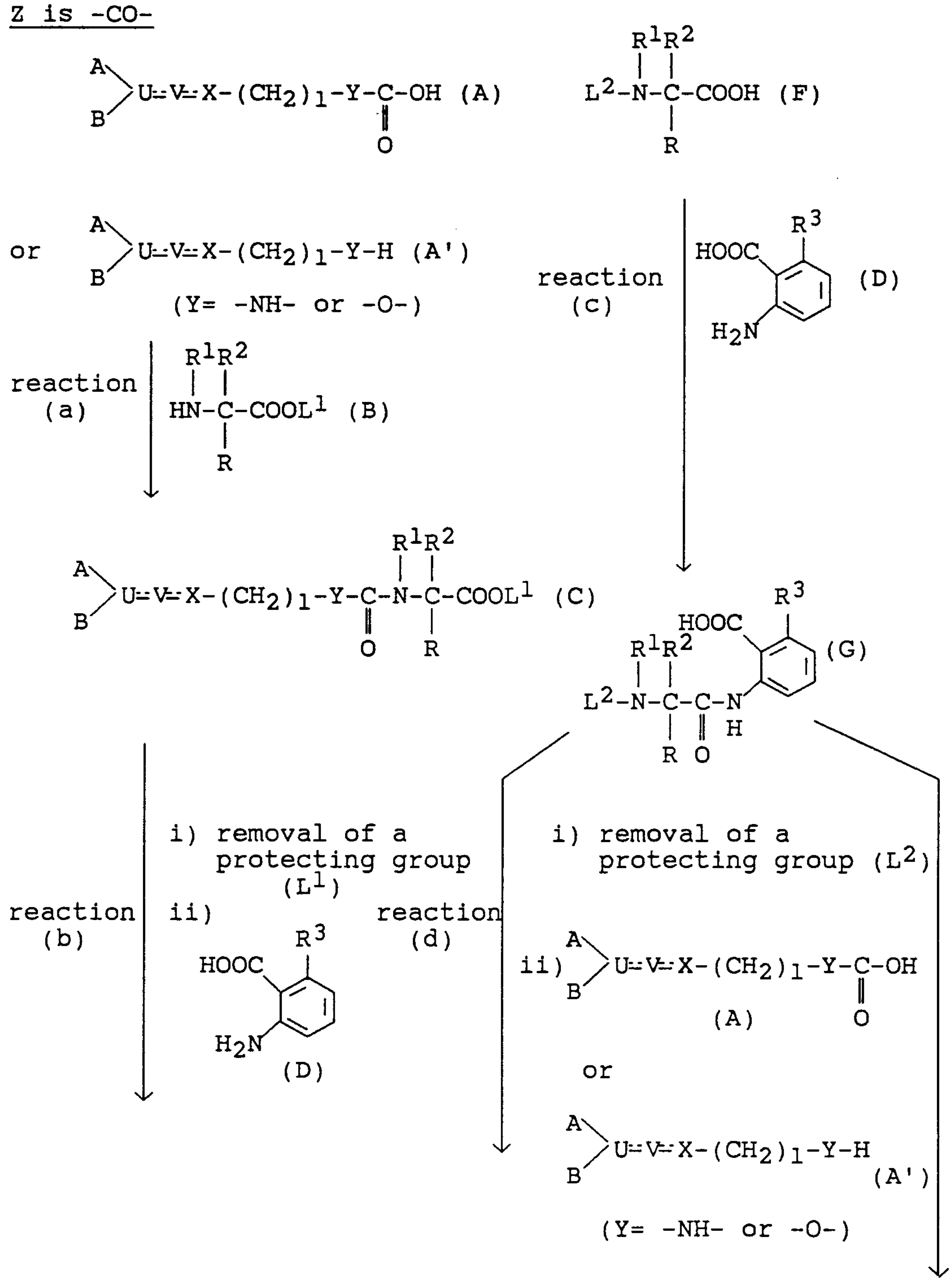


(continued)

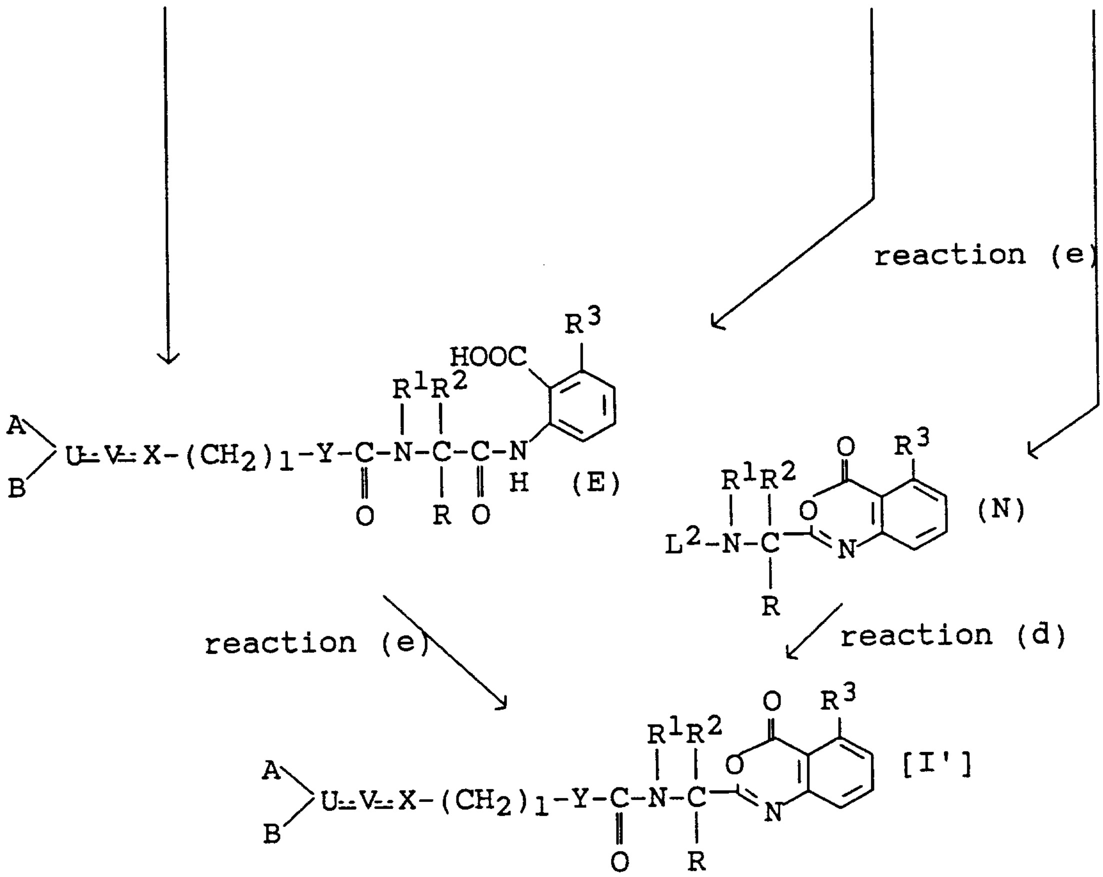
reaction (e)
(E)
(N)
reaction (e)
reaction (d)
[I']

flow-2

Z is $-CH_2-$ and Y is neither -NH- nor -O-

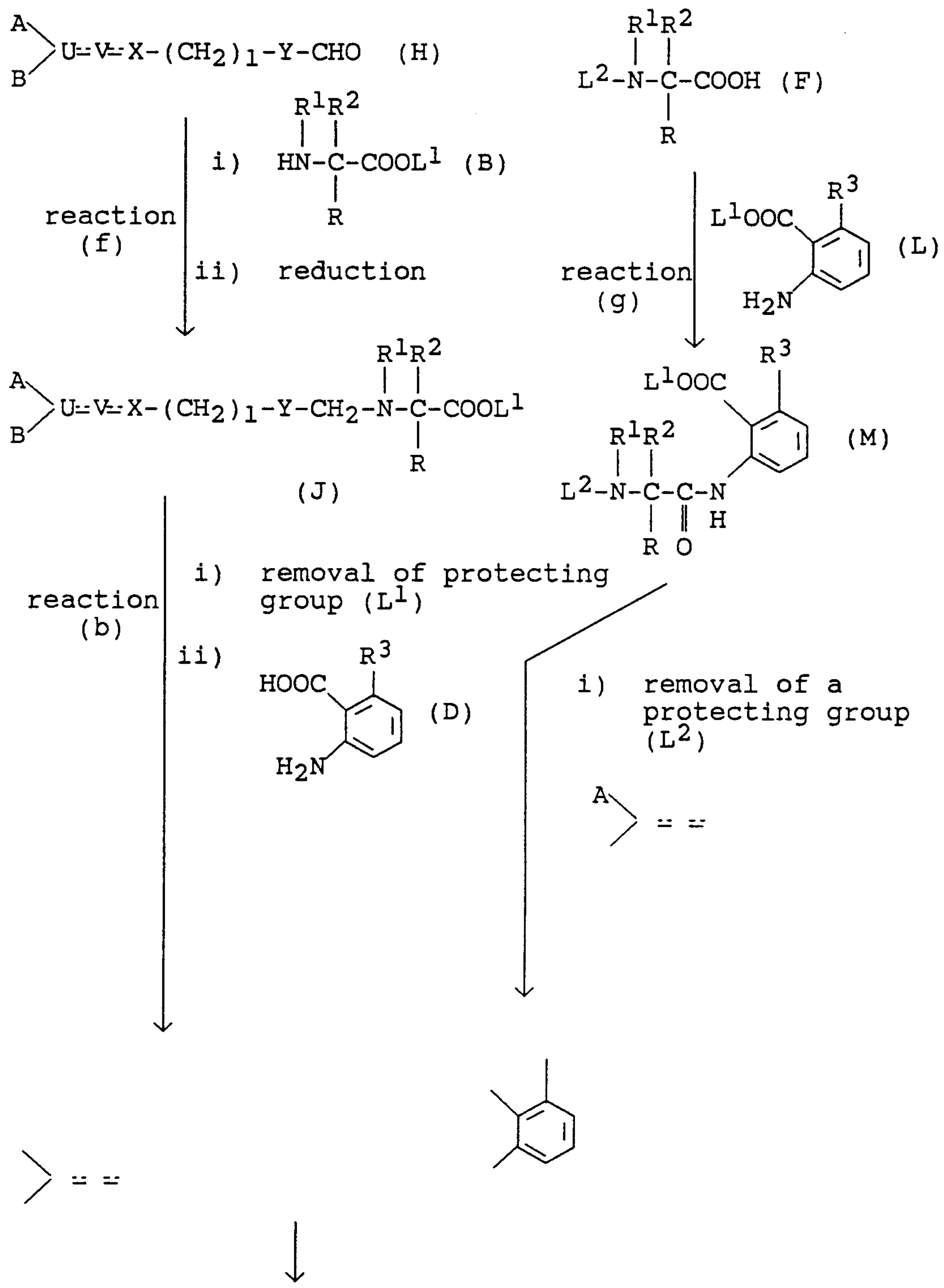

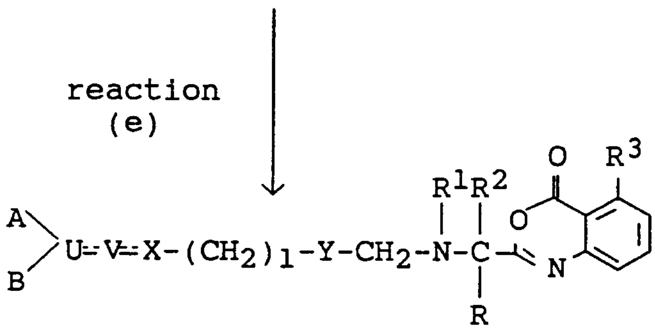


[I"]

flow-3

Z is -CO-, Y is $-CH_2-$ or means that $(CH_2)_l$ is directly bond to N

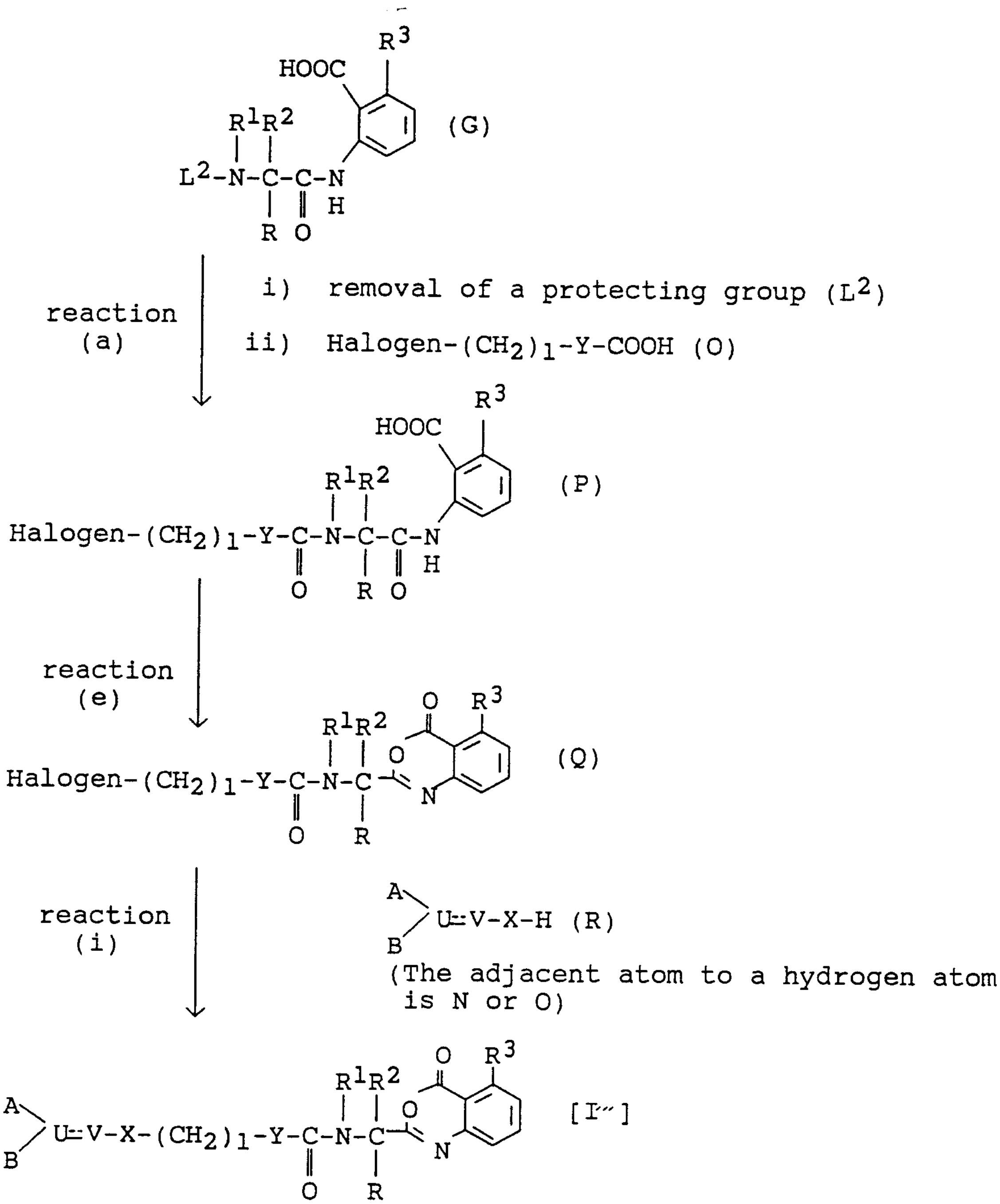


Hereinafter, each of the processes included in the above-described flows 1 to 3 will be described in detail.

The case in which Z is -CO-, will be described in more detail according to a flow 1. In a reaction (a), a compound (A) or (A') is subjected to a condensation reaction with an amino acid derivative (B), a carboxyl group of which is protected if necessary, thereby obtaining a compound (C). As examples of a preferable condensation reaction in this step, the following method (1) or (2) may be applied when a compound (A) is used as a starting material.

(1) The compound (A) is reacted with a carbonic acid monoalkyl ester such as isobutyl chloroformate in an inert organic solvent in the presence of a tertiary amine such as triethylamine or N-methylmorpholine, thereby obtaining a mixed acid anhydride. Then, the mixed acid anhydride, without being separated from the reaction mixture, is subjected to a condensation reaction with an amino acid derivative (B), thereby obtaining a compound (C).

(2) The compound (A) is reacted with N-hydroxysuccinimide in an inert organic solvent in the presence of a dehydrating-condensing agent such as water-solble carbodiimide hydrochloride, e.g., 1-ethyl-3-(3-dimethylaminopropyl)carbodiimide hydrochloride, or N,N'-dicyclohexylcarbodiimide, thereby obtaining a N-hydroxysuccinimide ester. Then, thus formed ester is reacted with a compound (B) in a mixed solvent such as a mixture of aceton and water in the presence of a base such as sodium carbonate, thereby obtaining a compound (C).

When a compound (A') is used, the compound (A') is reacted with phosgene, trichloromethyl chloroformate, carbonyldiimmidazole, or the like in a suitable solvent such as 1,4-dioxane or tetrahydrofuran in the presence of a tertiary amine such as triethylamine in a temperature of -20 °C to room temperature. Then, the resultant mixture is reacted with the compound (B), thereby obtaining a compound (C).

When $L^1$ of the compound (B) is a carboxyl-protecting group, the compound (C) can be synthesized by a condensation reaction using, for example, N,N'-dicyclohexylcarbodiimide in an inert organic solvent in the presence or absence of a suitable additive such as 1-hydroxybenzotriazole.

When $L^1$ is a carboxyl-protecting group in a reaction (b), $L^1$, that is, the carboxyl-protecting group of the compound (C) is removed by a conventional method, thereby obtaining a compound having a free carboxyl group. Then, the compound is subjected to a condensation reaction with an amino compound (D) in the same manner as in the reaction (a), thereby obtaining a compound (E).

Alternatively, the compound (E) can be synthesized via reactions (C) and (D). As shown in a reaction (c), the compound (D) having a free amino group is subjected to a condensation reaction with an amino acid derivative (F) having a free carboxyl group in the same manner as in a reaction (a), thereby synthesizing a compound (G). Then, the amino-protecting group $L^2$ of a compound (G) is removed by a conventional method according to a reaction (d), thereby preparing a compound having a free amino group. The carboxyl group of a compound (A) is activated in the same manner as in a reaction (a) and subjected to a condensation reaction with the above-described compound having a free amino group, thereby obtaining a compound (E). When a compound (A') is used in a reaction (d), a desired compound (E) can be obtained by using phosgene, trichloromethyl chloroformate or carbonyldiimidazole in the same manner as in a reaction (a).

In a reaction (e), the above-obtained compound (E) is cyclized by a dehydration-condensation reaction in an inert organic solvent in the presence of a condensing agent such as water-soluble carbodiimide hydrochloride, N,N'-dicyclohexylcarbodiimide, thereby obtaining a desired compound represented by a formula [I'].

In the above-described process, the ring closure reaction to form a benzoxazinone ring is performed in the final reaction step (e). Alternatively, the desired final compound represented by a formula [I'] can be obtained by subjecting a compound (G) to a ring closure reaction in advance in the same manner as in a reaction (e), thereby obtaining a benzoxazinone (N), followed by a reaction with a compound (A) or (A') according to the method described in a reaction (d).

Hereinafter, when Z is $-CH_2-$, each process in the flow 2, will be described in more detail.

In a reaction (f), a reductive alkylation reaction is carried out using an aldehyde (H) and an amino acid derivative (B), thereby obtaining a compound (J). As a preferable reductive alkylation reaction, there may be mentioned the following methods. A compound (H) and a compound (B) are dissolved in a solvent consisting of water, methanol, 1,4-dioxane, or the like alone or in combination, then proceeded a reaction in a weakly acidic condition, preferably in the pH range of 5 to 7, at a temperature of -30 °C to room temperature, preferably 0 °C to room temperature, thereby obtaining an imine. Then, the imine thus formed is subjected to a catalytic reduction in the presence of a platinum catalyst, or reduced using a reducing agent such as sodium borohydride or sodium cyanoborohydride, thereby obtaining a compound (J).

When $L^1$ is a carboxyl-protecting group, the carboxyl-protecting group $L^1$ of the compound (J) is removed by a known method and subjected to a condensation reaction with an amino compound (D) in the same manner as in the above-mentioned reaction (b), thereby obtaining a compound (K).

Alternatively, a compound (K) can be synthesized via reactions (g) and (h). As shown in a reaction (g), an amino acid derivative (F) having a free carboxyl group and an amino compound (L) are subjected to a condensation reaction in the same manner as in the above-described reaction (a), thereby obtaining a compound (M). Then, according to a reaction (h), an amino-protecting group $L^2$ is removed from the compound (M) by a known method, thereby obtaining a compound having a free amino group. Thus

obtained compound is subjected to a reductive alkylation reaction with an aldehyde (H) in the same manner as in a reaction (f). When $L^1$ of the obtained compound is a carboxyl-protecting group, the carboxyl-protecting group is removed by a known method, thereby obtaining a compound (K).

When $L^1$ of a compound (L) is a carboxyl-protecting group, the compound (L) and a compound (F) are condensed by using an N,N'-dicyclohexylcarbodiimide as a condensing agent in an inert organic solvent in the presence or absence of a suitable additive such as 1-hydroxybenzotriazole, N-hydroxy-5-norbornene-2,3-dicarboximide, thereby also obtaining a compound (M).

Thus obtained compound (K) is subjected to a dehydration-condensation reaction in the same manner as in the above-described reaction (e), thereby obtaining the desired compound of the present invention represented by a formula [I''].

Hereinafter, when Z is -CO-, Y is $-CH_2-$ or means that $(CH_2)_l$ is directly bond to N, each process, will be described in more detail according to the flow 3.

An amino-protecting group $L^2$ of a compound (G) obtained in the flow 1 is removed by a known method. Thus obtained compound having a free amino group is subjected to a condensation reaction with a compound (O) in the same manner as in the above-described reaction (a), thereby obtaining a compound (P). Then, the obtained compound (P) is subjected to ring closure reaction with dehydration in the same manner as in the above-described reaction (e), thereby obtaining a compound (Q). As shown in a reaction (i), the compound (Q) is reacted with a compound (R) in an inert organic solvent in the presence of a base such as sodium hydride, at a temperature of -20 °C to room temperature, thereby obtaining the desired compound of the present invention represented by a formula [I'''].

The inert organic solvent to be used in each reaction can be appropriately selected from the group consisting of N,N-dimethylformamide, tetrahydrofuran, 1,4-dioxane, 1,2-dimethoxyethane, methylene chloride, ethyl acetate, or the like.

After the completion of the reaction, in order to isolate and purify a desired compound from the reaction mixture, a known method in this art such as solvent extraction, column chromatography, recrystallization can be properly selected and used.

The compounds (A), (A'), (B), (D), (F), (H), (L), (O), and (R) used as a starting material are commercially available or can be easily derived or synthesized from known precursors using known methods.

A pharmaceutically acceptable salt can be produced from the benzoxazinone derivative of the present invention represented by a formula [I] produced by the above-mentioned method. Examples of a salt include an acid-addition salt with an inorganic acid such as hydrochloric acid, sulfuric acid, phosphoric acid, hydrobromic acid, and that with an organic acid such as tartaric acid, maleic acid, fumaric acid, succinic acid, sulfonic acid. Any of these salts is useful as an anti-inflammatory agent, an agent for suppressing neutrophile infiltration or a serine protease inhibitor.

Asymmetric carbon atoms could be included in the benzoxazinone derivative of the present invention represented by a formula [I]. If asymmetric carbon atoms are present, there may be present stereoisomers such as enantiomers and diastereomers based on these asymmetric carbon atoms. Moreover, there may be present geometric isomers such as a cis- or a trans-isomer. Accordingly, these all isomers and mixtures thereof should be included in the scope of the present invention.

The benzoxazinone derivertive represented by a formula [I] and salts thereof can be used as an effective ingredient of medicines. The medicine containing the benzoxazinone derivative or a salt thereof is effective as a prophylactic or therapeutic agent for various non-specific inflammatory diseases such as chronic arthritis, which is an inflammatory disease caused by abnormality of immunity; diseases of respiratory organs such as chronic obstructive pulmonary disease and pulmonary bronchitis based on chronic respiratory tract infection, adult respiratory distress syndrome, bronchi-obstructive type asthma classified as the adult type asthma; colon disease which is one of intestinal disease; and psoriasis which is one of dermatitis. The particular medicine is also effective as a prophylactic or therapeutic agent for the destruction and deterioration of tissue caused by elastase and various diseases including pancreatitis, nephritis, arteriosclerosis and septicemia.

The medicine containing the benzoxazinone derivative of the present invention represented by a formula [I] and a salt thereof as an effective ingredient can be administered by a suitable administration method such as orally, non-orally. It is also possible to administer a medicine via respiratory tract depending on the symptom of the diseases.

For oral administration, it may be in the form of a pellet, a capsule, granules, a powder, a liquid, and the like. For non-oral administration, it may be the form of a injection, a suppository, an ointment, a liquid, and the like. In preparing these forms for administration, an excipient, a binder, a collapsing agent, or other additives can be used according to a suitable method of the art. In particular, when administered via respiratory tract, the medicine can be administered in the form of spray such as an aerosol by the use of a

suitable surfactant or a propellant, etc.

When a medicine containing benzoxazinone derivatives of the present invention represented by a formula [I] and a salt thereof as an active ingredient is administered to a patient, the dose per day for an adult is usually regulated to be 1 to 100 mg in terms of the benzoxazinone derivative represented by a formula [I]. However, the dose is flexibly adjusted depending on the age, sex, weight, and the significance of a diseases, as well as administration style. The above should not be construed as limiting the scope of the present invention.

Hereinafter, the present invention will be described in more detail with reference to the following Examples and Working Examples, which should not be construed as limiting the scope of the present invention unless departing from the gist thereof.

Example 1

Synthesis of 2-[1(S)-[(2,2-diphenylethoxy-carbonyl )amino]-2-methylpropyl]-5-methyl-4H-3,1-benzoxazin-4-one (compound 1).

(1) 2-[(N-Benzyloxycabonyl-L-valyl)amino]-6-methylbenzoic acid

Acetone (18 ml) and N-benzyloxycarbonyl-L-valine N-hydroxysuccinimide ester (8.02g) were added to an aqueous solution (18 ml) of 2-amino-6-methylbenzoic acid (3.15g) and sodium carbonate (2.45g). The mixture was stirred at room temperature for 5 hours, acidified with 1N hydrochloric acid, followed by extraction with ethyl acetate. The organic layer was washed with 1N hydrochloric acid and saturated sodium chloride solution successively, dried over anhydrous sodium sulfate and concentrated. The residue was purified by silica gel column chromatography (an eluent: methylene chloride-ether) to give the above-captioned compound (3.71g).

(2) 6-Methyl-2-(L-valylamino)benzoic acid

2-[(N-benzyloxycarbonyl-L-valyl)amino]-6-methylbenzoic acid (1.21g) was dissolved in a mixture of methanol (99 ml) and water (1 ml), and 10% Pd-C (200 mg) was added. The mixture was stirred for 3 hours at room temperature under a hydrogen atmosphere. The Pd-C was filtered off and the filtrate was concentrated to give the above-captioned compound (830 mg).

(3) 2-{[N-(2,2-Diphenylethoxycarbonyl)-L-valyl]amino)-6-methylbezoic acid

Triethylamine (2.8 μl) and 2,2-diphenylethanol (0.4g) were added to a solution of trichloromethyl chloroformate (0.24 ml) in toluene (5 ml) under an ice-cooling and was stirred for 10 minutes. The reaction mixture was stirred at a temperature of 5 to 10°C for one hour followed by at room temperature for one hour, and toluene was distilled off under reduced pressure. A solution of the residue in tetrahydrofuran (6 ml) was added dropwise to a solution N-methylmorpholine (212 μl) and 6-methyl-2-(L-valylamino)benzoic acid (226 mg) in N,N-dimethylformamide (15 ml). After stirring for 20 hours at room temperature, the mixture was poured into 1N hydrochloric acid and extracted with ethyl acetate. The organic layer was washed with 1N hydrochloric acid and saturated sodium chloride solution successively, dried over anhydrous magnesium sulfate, and concentrated. The residue was purified by silica gel column chromatography (an eluent: ethyl acetate-hexane-acetic acid) to give the above-captioned compound (42 mg).

(4) 2-{1(S)-[(2,2-Diphenylethoxycarbonyl)amino]-2-methylpropyl}-5-methyl-4H-3,1-benzoxazin-4-one

Water-soluble carbodiimide hydrochloride (20 mg) was added to a solution of 2-{[N-(2,2-diphenylethoxycarbonyl)-L-valyl]amino}-6-methylbenzoicacid (42 mg) in N,N-dimethylformamide (2 ml) under an ice-cooling and the mixture was stirred for 15 hours. The mixture was concentrated under reduced pressure and the residue was purified by silica gel column chromatography (an eluent: methylene chloride-ether) to give the above-captioned compound (33 mg).

Example 2

Synthesis of 2-[1(S)-[[(diphenylmethoxy)acetyl]amino]-2-methylpropyl]-5-methyl-4H-3,1-benzoxazin-4-one (compound 2)

(1) (Diphenylmethoxy)acetic acid ethyl ester

Sodium hydride (60% dispersion in oil, 960 mg) was added to solution of diphenylmethanol (3.68g) and ethyl bromoacetate (3 ml) in N,N-dimethylformamide (30 ml) under an ice-cooling and the mixture was stirred for 2 hours. The resultant mixture was poured into ice-cold ammonium chloride solution and extracted with ether. The organic layer was washed with saturated sodium chloride solution, dried over anhydrous magnesium sulfate and concentrated. The residue was purified by silica gel column chromatography (an eluent: methylene chloride-ether) to give the above-captioned compound (4.62g).

(2) (Diphenylmethoxy)acetic acid

To a solution of (diphenylmethoxy)acetic acid ethyl ester (4.62g) in ethanol (80 ml) was added dropwise 1N sodium hydroxide (22.5 ml) under an ice-cooling over 20 minutes period and the mixture was stirred at room temperature for 8 hours. After concentrating under reduced pressure, the residue was dissolved in water and washed with ether. The water layer was acidified with 5% hydrochloric acid under an ice-cooling and extracted with ether. The organic layer was dried over anhydrous magnesium sulfate and concentrated to give the above-captioned compound (3.60g).

(3) 2-[[N-[(Diphenylmethoxy)acetyl]-L-valyl]amino]-6-methylbenzoic acid

N-methylmorpholine (0.19 ml) and isobutyl chloroformate (0.22 ml) were added to a solution of (diphenylmethoxy)acetic acid (407 mg) in tetrahydrofuran (15 ml) at -78°C. After the reaction mixture was stirred for 1.5 hours, 6-methyl-2-(L-valylamino)benzoic acid (300 mg) prepared in Example 1-(2) and a solution of N-methylmorpholine (0.17 ml) in N,N-dimethylformamide (18 ml) were added dropwise and stirred for 30 minutes. The resultant solution was stirred at room temperature for 17 hours, poured into ice-cold 1N hydrochloric acid and extracted with ethyl acetate. The organic layer was washed with 1N hydrochloric acid and saturated sodium chloride solution successively, dried over anhydrous magnesium sulfate and concentrated. The residue was purified by silica gel column chromatography (an eluent: ethyl acetate-hexane-acetic acid) to give the above-captioned compound (282 mg).

(4) 2-[1(S)-[[(Diphenylmethoxy)acetyl]amino]-2-methylpropyl]-5-methyl-4H-3,1-benzoxazin-4-one

2-[[N-[(Diphenylmethoxy)acetyl]-L-valyl]amino]-6-methylbenzoic acid (500 mg) was subjected to a ring closure reaction with dehydration in the same procedure as in Example 1-(4) to give the above-captioned compound (340 mg).

Example 3

Synthesis of 5-methyl-2-[2-methyl-1(S)-[[[(±)-α-(2-pyridyl)benzyloxy]acetyl]amino]propyl]-4H-3,1-benzoxazin-4-one (compound 3)

(1) (±)-α-(2-Pyridyl)benzyl alcohol

Sodium borohydride (2.27g) was added to a solution of 2-benzoylpyridine (5.50g) in ethanol (200 ml) under an ice-cooling and the mixture was stirred for 2.5 hours. Concentration gave a residue to which was added water, ammonium chloride and sodium chloride, and then the mixture was extracted with ether. The organic layer was dried over anhydrous magnesium sulfate and concentrated. The residue was purified by silica gel column chromatography (an eluent: methylene chloride-ether) to give the above-captioned compound (5.50g).

(2) [(±)-α-(2-Pyridyl)benzyloxy]acetic acid tert-butyl ester

Sodium hydride (60% dispersion in oil, 294 mg) was added to a solution of (±)-α-(2-pyridyl)benzyl alcohol (1.24g) and tert-butyl bromoacetate (0.97 ml) in N,N-dimethylformamide (15 ml) under an ice-cooling and the mixture was stirred at 1.5 hours. The resultant solution was poured into 5% citric acid solution and extracted with ether. The organic layer was washed with saturated sodium chloride solution, dried over anhydrous magnesium sulfate and concentrated. The residue was purified by silica gel column chromatography (an eluent: methylene chloride-ether) to give the above-captioned compound (2.05g).

(3) [(±)-α-(2-Pyridyl)benzyloxy]acetic acid hydrochloride

Trifluoroacetic acid (10 ml) was added to a solution of [(±)-α-(2-pyridyl)benzyloxy]acetic acid tert-butyl ester (2.05g) in methylene chloride (10 ml) under an ice-cooling and the mixture was stirred at 14 hours at room temperature. The resultant mixture was concentrated to dryness under reduced pressure. To the residue was added 4N hydrochloric acid-1,4-dioxane (10 ml) and the mixture was allowed to stand for 0.5 hour. Concentration of the mixture to dryness under reduced pressure gave the above-captioned compound (1.91g).

(4) 2-{1(S)-[(tert-Butoxycarbonyl)amino]-2-methylpropyl}-5-methyl-4H-3,1-benzoxazin-4-one

Water-soluble carbodiimide hydrochloride (1.34g) was added to 30 ml of N,N-dimethylformamide solution containing 2-{[N-(tert-butoxycarbonyl)-L-valyl]amino}-6-methylbenzoic acid (1.89g) prepared from N-(tert-butoxycarbonyl)-L-valine N-hydroxysuccinimide ester in the same procedure as in Example 1-(1) under an ice-cooling. The reaction mixture was stirred at room temperature for 18 hours and then concentrated under reduced pressure. The residue was purified by silica gel column chromatography (an eluent: methylene chloride-ether) to give the above-captioned compound (1.52g).

(5) 5-Methyl-2-[2-methyl-1(S)-[[[(±)-α-(2-pyridyl)benzyloxy]acetyl]amino]propyl]-4H-3,1-benzoxazin-4-one

Trifluoroacetic acid (14 ml of 35% solution in methylene chloride) was added to 2-{1(S)-[(tert-butoxycarbonyl)amino]-2-methylpropyl}-5-methyl-4H-3,1-benzoxazin-4-one (332 mg) under an ice-cooling and the mixture was stirred for 1.5 hours. Concentration of the mixture to dryness under reduced pressure gave 2-[1(S)-amino-2-methylpropyl]-5-methyl-4H-3,1-benzoxazin-4-one trifluoroacetate. On the other hand, N-methylmorpholine (0.3 ml) and of isobutyl chloroformate (0.17 ml) were added successively to a solution of (±)-α-(2-pyridyl)benzyloxy]acetic acid (349 mg) in tetrahydrofuran (10 ml) at -78°C and the mixture was stirred for 40 minutes. After addition of triethylamine (0.17 ml) dropwise, 10 ml of tetrahydrofuran solution of 2-[1(S)-amino-2-methylpropyl]-5-methyl-4H-3,1-benzoxazin-4-one trifluoroacetate prepared in above reaction was added, and the reaction mixture was stirred for 0.5 hours. After stirring at room temperature for 14 hours, the mixture was concentrated under reduced pressure. The residue was purified by silica gel column chromatography (an eluent: methylene chloride-ether) to give the above-captioned compound (183 mg).

Example 4

Synthesis of 2-[1(S)-[[[di-2-pyridyl)meth-oxy]acetyl]amino]-2-methylpropyl]-5-methyl-4H-3,1-benzoxazin-4-one (compound 4)

(1) (Di-2-pyridyl)methanol

Di-2-pyrydyl ketone (4.00g) was reduced with sodium borohydride in the same manner as in Example 3-(1), thereby obtaining the above-captioned compound (3.92g).

(2) [(Di-2-pyridyl)methoxy]acetic acid tert-butyl ester

The above-captioned compound (1.84g) was obtained in the same manner as in Example 3-(2) except that (di-2-pyridyl)methanol (2.05g) was used in place of (±)-α-(2-pyridyl)benzyl alcohol.

(3) [(Di-2-pyridyl)methoxy]acetic acid hydrochloride

The reaction was performed in the same manner as in Example 3-(3) except that [(di-2-pyridyl)-methoxy]acetate tert-butyl ester (1.80g) was used in place of [(±)-α-(2-pyridyl)benzyloxy]acetic acid tert-butyl ester, thereby obtaining the above-captioned compound (1.90g).

(4) 2-[1(S)-[[[(2-pyridyl)methoxy]acetyl]amino]-2-methylprophy]-5-methyl-4H-3,1-benzoxazin-one

2-[1(S)-Amino-2-methylpropyl]-5-methyl-4H-3,1-benzoxazin-4-one trifluoroacetate, prepared from 2-{1-(S)-[(tert-butoxycarbonyl)amino]-2-methylpropyl}-5-methyl-4H-3,1-benzoxazin-4-one (340 mg) in the same manner as in Example 3-(5), and [(di-2-pyridyl)methoxy]acetic acid hydrochloride (340 mg) were dissolved in methylene chloride (10 ml). To the mixture were added N-methylmorpholine (0.34 ml), 1-hydroxybenzotriazol (151 mg) and water-soluble carbodiimide hydrochloride (212 mg) under an ice-cooling and stirred for 3 hours. After stirring at room temperature for 15 hours, the reaction mixture was concentrated under reduced pressure. The residue was purified by silica gel column chromatography (an eluent: methylene chloride-ether) to give the above-captioned compound (136 mg).

Example 5

Synthesis of 2-{1(S)-[(benzyloxyacetyl)amino]-2-methylpropyl}-5-methyl-4H-3,1-benzoxazin-4-one (compound 5)

(1) Benzyloxyacetic acid ethyl ester

To a suspension of sodium hydride (60% dispersion in oil, 1.33g) in N,N-dimethylformamide (45 ml) was added benzyl alcohol (3.10 ml). The reaction mixture was stirred at room temperature for 0.5 hours, and then ethyl bromoacetate (3.70 ml) was added dropwise. After stirring for one hour, the reaction mixture was poured into ice-cold water and extracted with ethyl acetate. The organic layer was washed with 1N hydrochloric acid, saturated sodium hydrogencarbonate solution and saturated sodium chloride solution successively dried over anhydrous magnesium sulfate, and concentrated to give the above-captioned compound (5.20g).

(2) Benzyloxyacetic acid

Ethyl benzyloxyacetate (5.20g) was hydrolyzed in the same manner as in Example 2-(2) to give the above-captioned compound (3.65g).

(3) 2-{[N-(Benzyloxyacetyl)-L-valyl]amino}-6-methylbenzoic acid

Benzyloxyacetic acid (838 mg) was dissolved in a mixture of N,N-dimethylformamide (0.04 ml) and methylene chloride (20 ml), and oxalyl chloride (0.56 ml) was added at room temperature. After stirring for one hour, the reaction mixture was concentrated under reduced pressure. A solution of the residue in 1,4-dioxane (5 ml) was added dropwise to 80 ml of 1,4-dioxane-water (1:1) solution containing 6-methyl-2-(L-valylamino)benzoic acid (1.00g) prepared in Example 1-(2) and sodium hydrogencarbonate (608 mg). After stirring at room temperature for 2 hours, the reaction mixture was poured into a mixture of 1N hydrochloric acid-saturated sodium chloride solution to be acidified and extracted with ethyl acetate. The organic layer was washed with 1N hydrochloric acid, saturated sodium hydrogencarbonate solution and saturated sodium chloride solution successively dried over anhydrous sodium sulfate, and concentrated to give the above-captioned compound (1.74g) as a crude product.

(4) 2-{1(S)-[(Benzyloxyacetyl)amino]-2-methylpropyl}-5-methyl-4H-3,1-Bezoxazin-4-one

2-{[N-(Benzyloxyacetyl)-L-valyl]-amino}-6-methylbenzoic acid (1.74g) was subjected to a ring closure reaction with dehydration in the same manner as in Example 1-(4), thereby obtaining the above-captioned compound (604 mg).

Example 6

Synthesis of 2-[1(S)-[[(1-adamantyl)methoxy-carbonyl]amino]-2-methylpropyl]-5-methyl-4H-3,1-benzoxazin-4-one (compound 6)

(1) 2-[[N-[(1-Adamantyl)methoxycarbonyl]-L-valyl]amino]-6-methylbenzoic acid

A solution of 1-adamantanemethanol (166 mg) and triethylamine (0.14 ml) in tetrahydrofuran (4 ml) was added dropwise to a solution of trichloromethyl chloroformate (60 µl) in tetrahydrofurn (4 ml) under an ice-cooling and the mixture was stirred at room temperature for 0.5 hours. The resultant solution was cooled to 0°C and a solution of 6-methyl-2-(L-valylamino)benzoic acid (249 mg) and N-methylmorpholine (0.25 ml) in N,N-dimethylformamide (17 ml) was added dropwise thereto. The mixture was stirred for 15 minutes under an ice-cooling and for 20 hours at room temperature, poured into ice-cold 5% hydrochloric acid solution and extracted with ethyl acetate. The organic layer was washed with 1N hydrochloric acid and saturated sodium chloride solution successively, dried over anhydrous magnesium sulfate and concentrated. The residue was purified by silica gel column chromatography (an eluent: ethyl acetate-hexan-acetic acid) to give the above-captioned compound (85 mg).

(2) 2-[1(S)-[[(1-Adamantyl)methoxy-carbonyl]amino]-2-methylpropyl]-5-methyl-4H-3,1-benzoxazin-4-one

2-[[N-[(1-Adamantyl)methoxycarbonyl]-L-valyl]amino]-6-methylbenzoic acid (75 mg) was subjected to a ring closure reaction with dehydration in the same manner as in Example 1-(4), thereby obtaining the above-captioned compound (70 mg).

Example 7

Synthesis of 2-{1(S)-[(N,N-diphenyl-glycyl)amino]-2-methylpropyl}-5-methyl-4H-3,1-benzoxazin-4-one (compound 7)

(1) N,N-Diphenylglycine

Sodium hydride (60% dispersion in oil, 0.77g) was added to a solution of diphenylamine (2.99g) and tert-butyl bromoacetate (2.6 ml) in N,N-dimethylformamide (30 ml) under an ice-cooling and the mixture was stirred for one hour. After stirring at room temperature for one hour, the reaction mixture was stirred at 60°C for 18 hours. The resultant mixture was poured into ice-cold 10% citric acid solution and extracted with ethyl acetate. The organic layer was washed with saturated sodium chloride solution, dried over anhydrous magnesium sulfate and concentrated under reduced pressure. Trifluoroacetic acid (20 ml) was added to a solution of the residue in methylene chloride (20 ml), and the mixture was stirred at room temperature for 15 hours. The reaction mixture was poured into ice-cold 1N sodium hydroxide, washed with ether under an alkaline condition and then the water layer was acidified with hydrochloric acid and extracted with ethyl acetate. The ethyl acetate layer was washed with saturated sodium chloride solution, dried over anhydrous magnesium sulfate and concentrated. The residue was purified by silica gel column chromatography (an eluent: ethyl acetate-hexane-acetic acid) to give the above-captioned compound (512 mg).

(2) 2-{1(S)-[(N,N-diphenylglycyl)amino]-2-methylpropyl}-5-methyl-4H-3,1-benzoxazin-4-one

Isobutyl chloroformate (0.15 ml) was added dropwise to a solution of N,N-diphenylglycine (265 mg) and N-methylmorpholine (0.13 ml) in tetrahydrofuran (10 ml) at -20°C and the mixture was stirred for 0.5 hour. After adding triethylamine (0.16 ml), a solution of 2-[1(S)-amino-2-methylpropyl]-5-methyl-4H-3,1-benzoxazin-4-one trifluoroacetate in tetrahydrofuran (10 ml) prepared from 2-{1(S)-[(tert-butoxycarbonyl)-amino]-2-methylpropyl}-5-methyl-4H-3,1-benzoxazin-4-one (342 mg) was added dropwise at -20°C and the mixture was stirred for 0.5 hours. After stirring at room temperature for 2 hours, a precipitated salt was filtered off by filtration with suction. The filtrate was concentrated and purified by silica gel column chromatography (an eluent: methylene chloride-ether) to give the above-captioned compound (175 mg).

Example 8

2-{1(S)-[(4,4-diphenylbutyryl)amino]-2-methylpropyl}-5-methyl-4H-3,1-benzoxazin-4-one (compound 8)

(1) 4,4-Diphenylcrotonic acid methyl ester

To a suspension of (carbomethoxymethylene)triphenylphosphorane (10.22g) in tetrahydrofuran (50 ml) was added dropwise a solution diphenylacetaldehyde (5.0g) in tetrahydrofurn (30 ml) at room temperature and stirred for 5 days. The reaction mixture was refluxed for 3.5 hours, and then concentrated. The residue was purified by silica gel column chromatography (an eluent: methylene chloride-hexane) to give the above-captioned compound (6.56g).

(2) 4,4-Diphenylbutyric acid

To a solution of methyl 4,4-diphenylcrotonate (2.00g) in ethanol (80 ml) was added 10% Pd-C (200 mg), and the mixture was stirred at room temperature for 40 hours under a hydrogen atmosphere. After removing the catalyst by filtration with suction, the filtrate was concentrated. The residue was dissolved in ethanol (80 ml), and 1N sodium hydroxide (10.5 ml) was added dropwise thereto under an ice-cooling and then stirred for 4 days. After concentration of the mixture under reduced pressure, the residue was dissolved in water and washed with ether. The aqueous layer was acidified with hydrochloric acid under an ice-cooling. The resultant precipitated crystal was collected by filtration and then recrystallized from ether-hexane to give the above-captioned compound (1.53g).

(3) 2-{1(S)-[(4,4-diphenylbutyryl)amino]-2-methylpropyl}-5-methyl-4H-3,1-benzoxazin-4-one

N-Methylmorpholine (0.13 ml) and hydroxybenzotriazol (184 mg) were added to 15 ml of a methylene chloride solution containing 4,4-diphenylbutyric acid (241 mg) and 2-[1(S)-amino-2-methylpropyl]-5-methyl-4H-3,1-benzoxazin-4-one trifuluoroacetate prepared from 2-{(1(S)-[(tert-butoxycarbonyl)amino]-2-methylpropyl}-5-methyl-4H-3,1-benzoxazin-4-one (333 mg) under an ice-cooling. After stirring for 5 minutes, water-soluble carbodiimide hydrochloride (230 mg) was added and the mixture was stirred for 2 days at room temperature. The resultant mixture was concentrated to dryness. The residue was purified by silica gel column chromatography (an eluent: methylene chloride-ether) to give the above-captioned compound (33 mg).

Example 9

5-Methyl-2-[2-methyl-1(S)-[[[[[phenyl(2-pyridyl)methylene]amino]oxy]acetyl]amino]propyl]-4H-3,1-benzoxazin-4-one(compound 9)

(1) [[[phenyl(2-pyridyl)methylene]amino]oxy]acetic acid ethyl ester

The above-captioned compound (722 mg) was obtained in the same manner as in Example 2-(1) by using phenyl 2-pyrydyl ketoxime (1.00g), ethyl bromoacetate ester (0.64 ml) and sodium hydride (60% dispersion in oil, 222 mg).

(2) [[[phenyl(2-pyrydyl)methylene]amino]oxy]acetic acid

Sodium hydroxide (3 ml of 1N solution) was added to a solution of [[[phenyl(2-pyrydyl)methylene]-amino]oxy]acetic acid ethyl ester (700 mg) in ethanol (50 ml) under an ice-cooling and the mixture was stirred at room temperature for 16 hours. After concentrating under reduced pressure, the residue was dissolved in water and washed with ether. The water layer was acidified with hydrochloric acid, and the precipitated crystals were filtered to give the above-captioned compound (556 mg).

(3) 5-methyl-2-[2-methyl-1(S)-[[[[[phenyl(2-pyrydyl)-methylene]amino]oxy]acetyl]amino]propyl]-4H--3,1-benzoxazin-4-one

[[[Phenyl(2-pyrydyl)methylene]amino]-oxy]acetic acid (256 mg) was condensed with 2-[1(S)-amino-2-methylpropyl]-5-methyl-4H-3,1-benzoxazin-4-one trifluoroacetate in the same manner as in Example 3-(5) to

give the above-captioned compound (286 mg).

Chemical structures and physicochemical properties of the compounds in the above-mentioned Examples of the specification of the present invention are shown in Table 1

Table 1

| | Structure | Properties |
|---|---|---|
| Compound 1 | 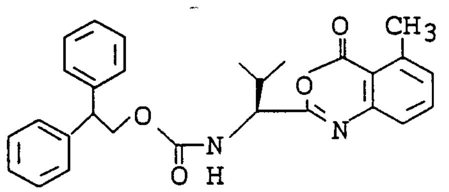 | Colorless amorphous |
| | $^1$HNMR ($CDCl_3$, δ value) | MS(m/z) |
| | 0.89(3H,d,J=6.8Hz),0.99(3H,d,J=6.8Hz),2.25(1H,m),2.79(3H,s),4.38(1H,t,J=7.5Hz),4.53(1H,dd,J=9.2,5.8Hz),4.60(1H,dd,J=11.0,7.5Hz),4.70(1H,dd,J=11.0,7.5Hz) 5.35(1H,d,J=9.2Hz),7.1-7.3(11H,m),7.37(1H,d,J=8.0Hz),7.63(1H,t,J=7.8Hz). | EI-MS: 456($M^+$) 259,216, 180,167, 160 |

| | Structure | Properties |
|---|---|---|
| Compound 2 | 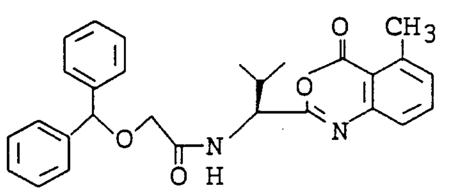 | Colorless amorphous |
| | $^1$HNMR ($CDCl_3$, δ value) | MS(m/z) |
| | 0.95(3H,d,J=6.8Hz),1.03(3H,d,J=6.8Hz),2.38(1H,m),2.80(3H,s),4.03(1H,d,J=15.4Hz),4.12(1H,d,15.4Hz),4.89(1H,dd,J=8.9,5.2Hz),5.53(1H,s),7.2-7.4(12H,m),7.56(1H,d,J=8.0Hz),7.62(1H,t,J=7.8Hz). | EI-MS: 457($MH^+$) 289,274, 259,231, 167,160 |

Table 1 (continued)

| | Structure | Properties |
|---|---|---|
| Compound 3 | 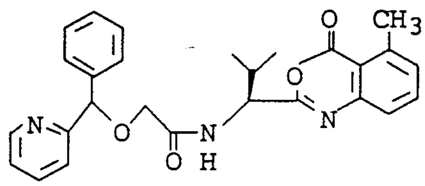 | Colorless amorphous |
| | $^1$HNMR ($CDCl_3$, δ value) | MS(m/z) |
| | 1.00,1.05,1.06&1.08(total 6H,d each,J=6.8Hz),2.43(1H,m),2.78&2.81(total 3H,s each),4.07,4.12,4.18&4.24(total 2H,d each,J=15.8 Hz),4.92(1H,dd,J=8.9,5.8Hz),5.62&5.70(total 1H,s each),7.2-7.7(11H,m),8.41&8.44(total 1H,d each,J=8.9Hz),8.60&8.69 (total 1H,d each,J=4.0Hz). | FAB-MS: 458(MH$^+$) |

| | Structure | Properties |
|---|---|---|
| Compound 4 | 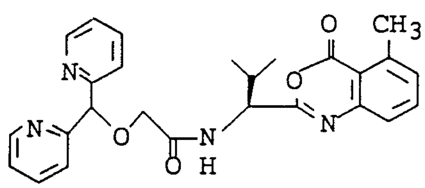 | Colorless oil |
| | $^1$HNMR ($CDCl_3$, δ value) | MS(m/z) |
| | 1.05(3H,d,J=6.7Hz),1.07(3H,d,J=6.7Hz),2.44(1H,m),2.79(3H,s),4.19(1H,d,J=15.9Hz),4.26(1H,d,J=15.9Hz),4.94(1H,dd,J=9.1,6.2 Hz),5.82(1H,s),7.2-7.4(5H,m),7.47(1H,d,J=7.8Hz),7.61(1H,t,J=7.8Hz),7.73(2H,td,J=7.7,1.5Hz),8.63(1H,d,J=4.3Hz),8.70 (1H,d,J=4.5Hz),8.88(1H,d,J=9.1 Hz). | FAB-MS: 459(MH$^+$) |

Table 1 (continued)

| | Structure | Properties |
|---|---|---|
| Compound 5 | 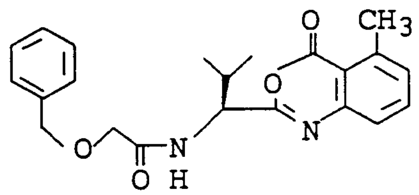 | White powdery |
| | $^1$HNMR ($CDCl_3$, δ value) | MS(m/z) |
| | 0.98(3H,d,J=6.8Hz),1.04(3H,d, J=6.8Hz),2.38(1H,m),2.80(3H,s), 4.03(1H,d,J=15.3Hz),4.13(1H,d, J=15.3Hz),4.64(1H,d,J=11.8Hz), 4.71(1H,d,J=11.8Hz),4.90(1H,dd, J=9.2,5.5Hz),7.3-7.4(8H,m), 7.63(1H,t,J=7.8Hz). | FAB-MS: 381($MH^+$) |

| | Structure | Properties |
|---|---|---|
| Compound 6 | 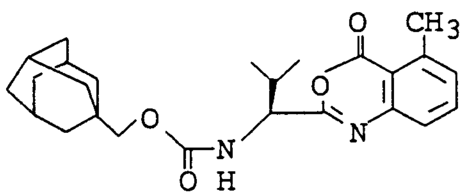 | colorless amorphous |
| | $^1$HNMR ($CDCl_3$, δ value) | MS(m/z) |
| | 0.98(3H,d,J=6.8Hz),1.05(3H,d, J=6.8Hz),1.4-2.0(15H,m),2.31 (1H,m),2.79(3H,s),3.71(2H,br.s), 4.59(1H,br.dd),5.40(1H,br.d), 7.30(1H,d,J=7.7Hz),7.43(1H,d, J=7.7Hz),7.64(1H,t,J=7.7Hz). | El-MS: 424($M^+$) 381,154 149 |

Table 1 (continued)

| | Structure | Properties |
|---|---|---|
| Compound 7 | 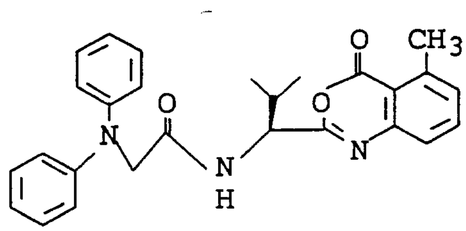 | Colorless amorphous |
| | $^{1}$HNMR ($CDCl_3$, δ value) | MS(m/z) |
| | 0.72(3H,d,J=6.9Hz),0.91(3H,d,J=6.9Hz),2.30(1H,m),2.78(3H,s),4.34(1H,d,J=18.2Hz),4.52(1H,d,J=18.2Hz),4.90(1H,dd,J=9.3,4.8Hz),7.0-7.3(13H,m),7.61(1H,t,J=7.8Hz). | FAB-MS: 442($MH^+$) |

| | Structure | Properties |
|---|---|---|
| Compound 8 | 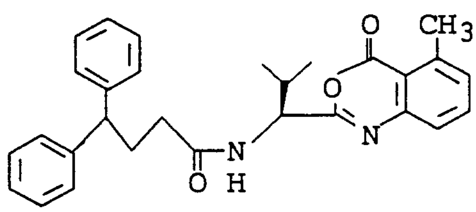 | Colorless amorphous |
| | $^{1}$HNMR ($CDCl_3$, δ value) | MS(m/z) |
| | 0.94(3H,d,J=6.8Hz),1.01(3H,d,J=6.8Hz),2.2-2.5(5H,m),2.79(3H,s),3.99(1H,t,J=7.9Hz),4.87(1H,dd,J=8.8,5.4Hz),6.02(1H,d,J=8.8Hz),7.1-7.3(11H,m),7.38(1H,d,J=7.9Hz),7.62(1H,t,J=7.9Hz). | FAB-MS: 455($MH^+$) |

Table 1 (continued)

| | Structure | Properties |
|---|---|---|
| Compound 9 | 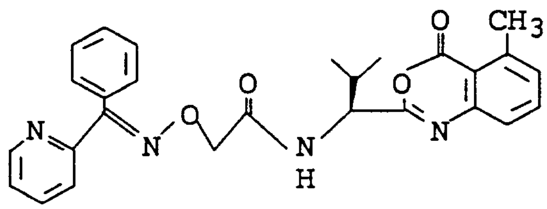 | Colorless amorphous |
| | $^{1}$HNMR ($CDCl_3$, δ value) | MS(m/z) |
| | 0.94(3H,d,J=6.8Hz),0.98(3H,d,J=6.8Hz),2.41(1H,m),2.79(3H,s),4.66(1H,d,J=16.2Hz),4.85(1H,d,J=16.2Hz),4.87(1H,dd,J=9.2,7.7Hz),7.25-7.5(9H,m),7.57(1H,t,J=7.8Hz),7.86(1H,td,J=7.7,1.8Hz),8.76(1H,d,J=9.2Hz),9.04(1H,br.d,J=4.9Hz). | FAB-MS: 471($MH^+$) |

These Example should not be construed as limiting the scope of the present invention. For example, the compounds shown in Table 2 fall within the present invention.

Table 2

| | Structure |
|---|---|
| Compound 10 | 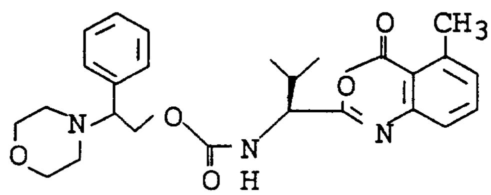 |

| | Structure |
|---|---|
| Compound 11 | 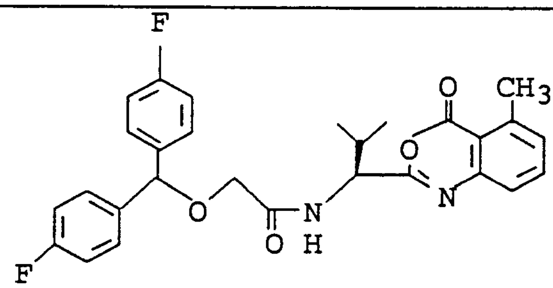 |

| | Structure |
|---|---|
| Compound 12 | 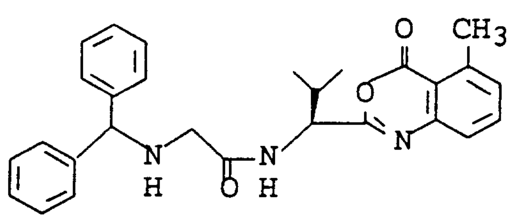 |

Table 2 (continued)

| | Structure |
|---|---|
| Compound 13 | 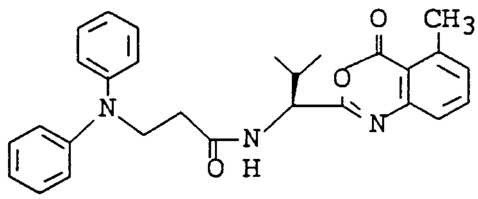 |

| | Structure |
|---|---|
| Compound 14 | 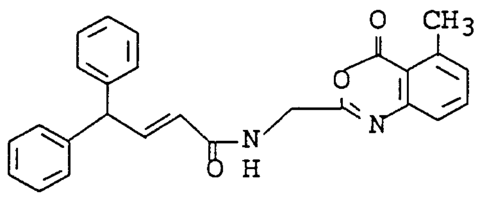 |

| | Structure |
|---|---|
| Compound 15 | 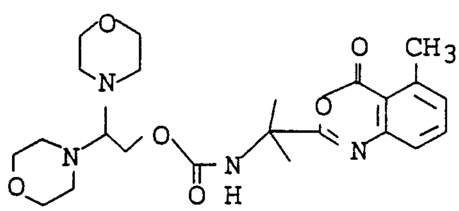 |

| | Structure |
|---|---|
| Compound 16 | 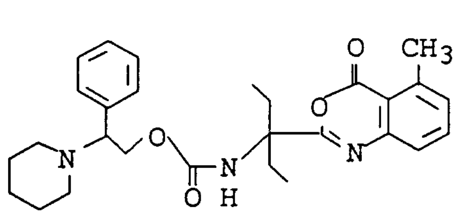 |

Table 2 (continued)

| | Structure |
|---|---|
| Compound 17 | 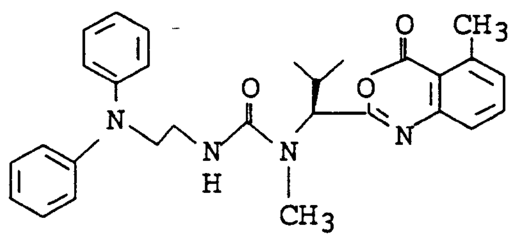 |

| | Structure |
|---|---|
| Compound 18 | 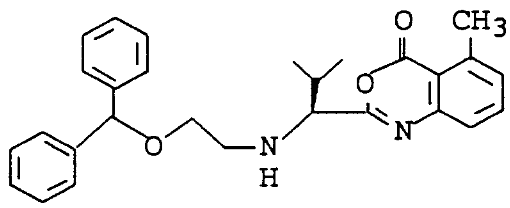 |

| | Structure |
|---|---|
| Compound 19 | 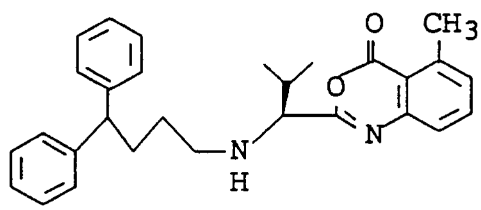 |

Benzoxazinone derivatives of the present invention represented by formula (I) was tested for the protease inhibiting activity, the activity for suppressing neutrophile chmotaxis, and the effect for suppressing carrageenin-induced air pouch inflammation. Hereinafter, we will describe results of the above mentioned tests.

Test 1: Serine Protease Inhibiting Activity

Activities of the Benzoxazine derivatives of the present invention against human leukocyte elastase (HLE), human sputum elastase (HSE), human cathepsin G (HCG), bovine pancreatic $\alpha$-chmotrypsin (CYT) and bovine pancreatic trypsin (TRY) were measured by the following method.

(1) Measurement of HLE Inhibitory Activity

a) Enzyme Preparing Method

A buffy coat was obtained by subjecting human vein blood to centrifugation. To the buffy coat was added distilled water to make a hypotonic solution, thereby bursting erythrocytes. To the residual blood cell component was added a 33.4% of Conrey 400 solution/9% Ficoll solution (the ratio of the former solution to the latter solution is 10:24), and the mixed solution was subjected to density gradient centrifugation, thereby obtaining neutrophiles. The neutrophile fraction was washed with Hanks' buffer for three times and then cells were recovered and cryopreserved at -80°C until use. The cryopreserved-cells were thawed with warm water at 37°C and suspended in a 9-fold volume of lysis buffer containing 0.1M Tris-HCl (pH7.5), 1M $MgCl_2$ and 0.1% Brij 35. The suspended cells are destroyed by means of polotoron with ice-cooling. The cell suspension was subjected to centrifugation at 100,000 x g for 1 hour. After removing precipitates, 2-fold volume of a Tris-HCl buffer containing 5 mM Tris-HCl(pH 7.5), 1M NaCl and 1.1% Brij 35 was added to the supernatant, thereby obtaining a crude solution of an enzyme extract. The crude solution was subjected to aprotinine affinity column and extracted with an elution buffer containing 50 ml of glycine-HCl (pH 3.3), 1M NaCl and 0.1% Brij 35, thereby obtaining an active fraction. The obtained active fraction was further subjected to MONO S-ion-exchange chromatography and eluted with 2M NaCl linear gradient. The obtained fraction was dialyzed against 25 mM Tris-HCl (pH8.6) and lyophilized, thereby obtained an enzyme sample.

b) Method for Evaluating Enzyme Inhibitory Activity

A test compound was dissolved in DMSO (for fluorometry) and prepared various concentration of the solution. To each concentration was added 2-fold volume of 0.2M Tris-HCl (pH8.6), thereby obtaining a test solution. Synthetic substrate (Funakoshi, L-1335) was dissolved in DMSO and 2-volume of 0.2M Tris-HCl (pH8.6) was added thereto, thereby obtaining a substrate solution. The reaction was performed using a microplate having 96 wells (Nunk). Six-time reactions were repeated for each concentration. One handred and forty µl of an enzyme solution containing 0.2M Tris-HCl (pH8.6), 30 µl of a test solution and 30 µl of a substrate solution per well of the microplate were respectively pre-incubated at 37°C for 10 minutes. After completion of the incubation, the enzyme solution was mixed with the test sample and incubated for 10 minutes and then the substrate solution was added to perform an enzyme reaction. The absorbance of released P-nitroanilide (PNA) was measured at the wave length of 405 nm by means of a microplate reader (MTP-100; CORONA), thereby determining the substrate decomposition. The rate of the substrate decomposition was shown as an average value of 6 repeated reactions. The inhibitory concentration of a test compound relative to the amount of the substrate decomposition in the reaction mixture without a test compound was regarded as an $IC_{50}$ value. The final concentration of HLE and a synthesized substrate in the reaction mixture were 5.63 nM and 0.2625 mM, respectively.

2) Measurement of HSE Inhibitory Activity

a) Enzyme obtaining route
HSE was obtained from Sigma Inc.

b) Method for Evaluating Enzyme Inhibitory Activity

The same method as used in the method for evaluating HLE inhibitory activity was used except that a buffer solution (pH 7.0) was used. The final concentration of HSE and synthetic substrate (Funakoshi, L-1335) in a reaction mixture were 10 nM, 0.25 mM, respectively.

3) Measurement of HCG Inhibitory Activity

a) Enzyme obtaining route
HCG was obtained from Cosmo Bio Inc.

b) Method for Evaluating Enzyme Inhibitory Activity

The same method as in the measurement of HLE inhibitory activity was used except that a buffer solution (pH 7.5) was used. The final concentration of HCG and synthetic substrate (Funakoshi, L-1010) in a

reaction mixture were 50 nM and 0.5 mM, respectively.

4) Measurement of CYT Inhibitory Activity

a) Enzyme obtaining route
CYT was obtained from Sigma Co., Ltd.

b) Method for Evaluating Enzyme Inhibitory Activity

Substantially the same method as used in the measurement of the HLE inhibitory activity was used except that a buffer solution (pH 8.0) was used. The final concentration of CYT and synthetic substrate (Funakoshi, L-1010) in a reaction mixture were 3.4 nM and 0.273 mM, respectively.

5) Measurement of TRY Inhibitory Activity

a) Enzyme obtaining route
TRY was obtained from Sigma Inc.

b) Method for Evaluating Enzyme Inhibitory Activity

The same method as in the measurement of the HLE inhibitory activity was used except that a buffer solution (pH 7.5) was used. The final concentration of TRY and synthetic substrate (Z-Arg-pNA; Funakoshi) in a reaction mixture were 25 nM and 1.0 mM, respectively.

6) Results

Results of 1) to 5) are shown in Table 3

Table 3

| Inhibitory activity of compounds on several kinds of proteases | | | | | |
|---|---|---|---|---|---|
| Compound | $IC_{50}$ value ($\mu$M) | | | | |
| | HLE | HSE | HCG | CYT | TRY |
| 1 | 0.07 | 0.28 | >12.5 | 1.40 | >12.5 |
| 2 | 0.22 | - | >27.4 | 0.83 | - |
| 3 | 0.34 | - | 58.3 | 2.08 | - |
| 4 | 0.55 | - | - | - | - |
| 6 | 0.21 | 0.26 | >25.0 | 1.55 | >12.6 |
| 7 | 0.79 | - | - | - | - |
| 8 | 0.40 | - | - | - | - |
| 9 | 0.55 | - | - | - | - |

As apparent from Table 3, the compound of the present invention represented by the above-mentioned formula [1] exhibited a strong inhibitory activity with a high selectivity against serine protease, particularly human neutrophile elastase.

Test 2: The Human Neutrophile Chemotaxis Suppressing Activity of the Invented Compound

The neutrophile fraction (60% percoal fraction having neutrophile purity of 90% or more) was taken from the human peripheral blood by means of percoal density gradient centrifugation at 2300 rpm for 20 minutes.

An intercell having a pore diameter of 5 µm, containing 8 x $10^5$ of neutrophiles and the compound of the present invention adjusted in various concentrations was placed in each well of a chemotaxis chamber (KURABO), in which has been added 1300 µl of a medium containing $10^{-7}$M of formylmethionyl-leucyl-phenylalanine (fMLP), which is a neutrophile chemotaxis factor. After incubating for one hour at 37°C in an incubator containing 5% carbon dioxide, the number of cells migrating from the upper side of the intercell membrane into the lower side of the membrane in the chamber was counted by the following method. That is, the lower side surface of the intercell membrane was washed with PBS for 4 times and fixed with methanol and dyed with hematoxylin-eosin solution. The membrane was removed from the intercell and placed on a slide, dried in air, followed by sealing with Canada balsam. The cell number was microscopically counted in 5 visual fields. Taking average number of the five fields, the average was employed as the number of migrating cells. The value subtracting the migrating cell number in a control experiment containing no fMLP from the cell number in an experiment containing no the compound of the present invention was regarded as 100, the chemotaxis suppressing activity was determined as the following formula:

Neutrophilic chemotaxis suppressing activity (%)
= 100 - A/B x 100
= 100 - (C - D)/(E-D) x 100

Where:
A: chemotaxis measured in the experiment containing the compound of the present invention;
B: chemotaxis measured in the experiment not containing the compound of the present invention
C: the number of migrating cells measured in the experiment containing both the compound of the present invention and fMLP.
D: the number of migrating cells in the experiment containing neither the compound of the present invention nor fMLP.
E: the number of migrating cells in the experiment containing only fMLP.
The result are shown in Table 4

Table 4

| Suppressive activity of compounds on the neutrophile chemotaxis | | | |
|---|---|---|---|
| Compound | Concentration (µM) | Chemotaxis | Chemotaxis suppressing activity (%) |
| 1 | 0<br>12.5 | 100<br>4.7 | -<br>95.3 |
| 2 | 0<br>0.125<br>1.000 | 100<br>70.6<br>57.4 | -<br>29.4<br>42.6 |

As apparent from Table 4, the compound of the present invention represented by a formula [1] exhibits a remarkable neutrophile chemotaxis suppressing activity.

Test 3 Suppressive Effect of the Invented Compound on Neutrophile Infiltration of Carrageenin-Induced Air Pouch Inflammation

SD female rats in 5 weeks old, having the weight in the range of 110 to 130g were used. A group was regarded to have 5 rats. In the first step, an air pouch was formed by a hypodermic injection of 8 ml of air on the back of a rat under ether anesthesia. Twenty four hours later, to the air pouch was added 5 ml of a saline containing 1% carrageenin (Wako Co., Ltd.). A test compound of the present invention, which has been dissolved in 200 µl DMSO (dimethylsulfoxide), was administered in the air pouch immediately after the carrageenin injection.

Five hours later, the rat was killed and the blood was released. Then, 5 ml of PBS containing EDTA (ethylenediamine tetraacetate) was injected in the air pouch. The exudate in the air pouch was washed and collected. An aliquot of the exudate was measured by a colter counter and determined the number of cells in the collected exudate.

The value subtracting migrating cell number in an experiment performed without carrageenin injection from the migrating cell number in a control experiment administering no the compound of the present invention was regarded as 100, the chemotaxis suppressing activity was determined as the following formula:

Cell Infiltration Suppressing Activity (%)
= 100 - S/T x 100
= 100 - (U - V )/(W - V) x 100

Wherein:

S: cell infiltration measured in the experiment administering the compound of the present invention;
T: cell infiltration measured in the experiment not administering the compound of the present invention
U: the number of infiltrated cells measured in the experiment administering both the compound of the present invention and carrageenin.
V: the number of infiltrated cells in the experiment administering neither the compound of the present invention nor carrageenin.
W: the number of infiltrated cells in the experiment administering only carrageenin.

The results are shown in Table 5

Table 5

| Suppressive effect of a compound on the Neutrophile Infiltration of Carrageenin-induced Air Pouch inflammation | | | |
|---|---|---|---|
| Compound | Dose (μmol) | Cell Infiltration | Rate (%) of Suppressing Cell Infietration |
| Control | 0 | 100 | - |
| 3 | 10.9 | 66.1 | 33.9 |

As apparent from Table 5, the compound of the present invention represented by a formula [1] exhibits a neutrophile infiltration suppressing activity in carrageenin-induced air pouch.

[Application in the Industrial Field]

The benzoxazinone derivative of the present invention is a novel compound and widely effects on the process of inflammation. Particularly, the compounds of the present invention exhibit excellent inhibitory activity on elastase. Also, the compounds permit suppressing the chemotaxis of human peripheral blood neutrophiles toward chemically attracting substance derived from bacteria. Further, the compounds of the present invention permits suppressing the neutrophile infiltration in an animal inflammatory model.

Under the circumstances, the compounds of the present invention are useful as an anti-inflammatory agent, an agent for suppressing neutrophile infiltration, or as a serine protease inhibitor, making it possible to use the compounds of the present invention as a medicine.

## Claims

**1.** A benzoxazinone derivative represented by the following formula [I] or a pharmaceutically acceptable acid-addition salt thereof:

(1) Formula

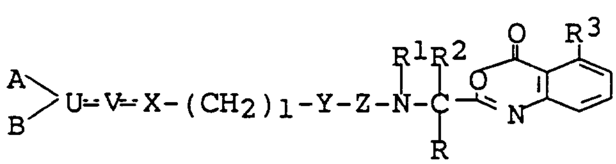

[I]

Wherein:

R is a hydrogen atom or a lower alkyl group;

$R^1$ is a hydrogen atom or a lower alkyl group;

$R^2$ is a hydrogen atom or a lower alkyl group;

$R^3$ is a hydrogen atom or a lower alkyl group;

A is a phenyl group which may be substituted with a halogen atom, or a heterocyclic ring, or adamantyl group;

B is a hydrogen atom, or phenyl group which may be substituted with a halogen atom or a heterocyclic ring:

U is
 >CH-,

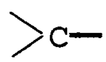

or >N-;

V is -O-, $-CH_2-$, =N-, -NH- or -CH=, or means that U is directly bond to X;

X is -O-, =CH- or $-CH_2-$, or means that V is directly bond to $(CH_2)_l$;

Y is - $CH_2$ -, -NH- or -O-, or means that $(CH_2)_l$ is directly bond to N;

Z is -CO- or $CH_2$-;

-----

is a double bond or a single bond;

l is an integer of 0 to 3.

**2.** An anti-inflammatory agent containing an effective amount of said compound according to claim 1 and a pharmaceutically acceptable carrier to exhibit an anti-inflammatory activity.

**3.** A neutrophile infiltration suppressing agent containing an effective amount of said compound according to claim 1 and a pharmaceutically acceptable carrier to exhibit a neutrophile infiltration suppressing activity.

**4.** A serine protease inhibiting agent containing an effective amount of said compound according to claim 1 and a pharmaceutically acceptable carrier to exhibit a serine protease inhibiting activity.

# INTERNATIONAL SEARCH REPORT

International application No.
PCT/JP92/01550

**A. CLASSIFICATION OF SUBJECT MATTER**

Int. Cl$^5$ C07D265/22, 413/12, 413/14, 417/12, A61K31/535

According to International Patent Classification (IPC) or to both national classification and IPC

**B. FIELDS SEARCHED**

Minimum documentation searched (classification system followed by classification symbols)

Int. Cl$^5$ C07D265/22, 413/12, 413/14, 417/12, A61K31/535

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | Biochem. Biophys. Res. Commun., Vol. 140, No. 3, (1986), R. W. Spencer et al. "Inhibition of Serine proteases by benzoxazinones : effects of electron withdrawal and 5-substitution" p. 928-933 | 1-4 |
| A | Chemical Abstracts, Vol. 107, No. 3, (1987), Abstract No. 19864j | 1-4 |

☐ Further documents are listed in the continuation of Box C. ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| February 15, 1993 (15. 02. 93) | March 9, 1993 (09. 03. 93) |
| Name and mailing address of the ISA/<br>Japanese Patent Office<br>Facsimile No. | Authorized officer<br><br>Telephone No. |